# FASCICULE DE BREVET EUROPEEN

(11) **EP 2 934 626 B1**
(45) Date de publication et mention de la délivrance du brevet: **11.07.2018**
(21) Numéro de dépôt: 13824602.0
(22) Date de dépôt: 13.12.2013
(51) Int. Cl.: A61M 5/14, A61J 1/10, A61M 5/315, A61J 1/20

(54) **SYSTÈME DE PERFUSION POUR L'ADMINISTRATION D'UNE SOLUTION ET LE RINÇAGE DUDIT SYSTÈME**
INFUSIONSSYSTEM ZUR VERABREICHUNG EINER LÖSUNG UND SPÜLUNG DIESES SYSTEMS
INFUSION SYSTEM FOR ADMINISTERING A SOLUTION AND RINSING OF SAID SYSTEM

(30) Priorité: 21.12.2012 FR 1262738
(43) Date de publication de la demande: 28.10.2015
(73) Titulaire: Maco Pharma, 59420 Mouvaux (FR)
(72) Inventeur: HANTUTE, Emeline, F-59290 Wasquehal (FR)
(74) Mandataire: Sayettat, Julien Christian
(86) Numéro de dépôt international: PCT/FR2013/053077
(87) Numéro de publication internationale: WO 2014/096643

(56) Documents cités:
- EP-A1- 1 722 840
- WO-A1-95/17134
- WO-A1-2005/087294
- US-A- 5 643 205
- US-A1- 2008 086 097

## Description

L'invention concerne un système de perfusion pour la perfusion d'une solution médicamenteuse à un patient.

Elle s'applique typiquement à la perfusion par gravité destinée à permettre l'administration parentérale d'une solution injectable à un patient. Dans un exemple particulier de réalisation, la solution à injecter contient un principe actif nécessitant un dosage précis, tel qu'un agent antimitotique ou antibiotique.

On connaît du document EP-1 060 754 un système de perfusion formant un système clos et unitaire comprenant au moins deux poches, l'une contenant une solution médicamenteuse et l'autre un soluté de rinçage, et des moyens de perfusion. Ce système de perfusion comprend en outre des moyens de sélection à commande externe aptes à sélectionner le passage dans les moyens de perfusion du soluté contenant le médicament et/ou du soluté de rinçage.

Toutefois, l'utilisation de ce système nécessite la présence du personnel soignant lors de la perfusion puisque ce personnel doit manoeuvrer les moyens de sélection à commande externe. En outre, si le personnel soignant n'arrête pas la perfusion de la solution médicamenteuse avant que la poche la contenant ne soit complètement vide, l'air contenu dans celle-ci peut être introduit dans les moyens de perfusion. Dans ce cas, lors du rinçage, le volume d'air est administré au patient, provoquant ainsi une embolie gazeuse.

Pour résoudre ces problèmes, le document FR-2 871 062 propose notamment une poche à usage médical pour perfusion d'un médicament par gravité, comportant au moins deux compartiments, l'un contenant une solution médicamenteuse et l'autre un soluté de rinçage, et des moyens de séparation/communication des compartiments ne permettant au soluté de rinçage de pénétrer automatiquement dans le compartiment contenant le médicament qu'en fin de perfusion. Dans une réalisation particulière, une valve à seuil de pression est placée entre les deux compartiments placés l'un au dessus de l'autre, de sorte que lorsque le compartiment du bas contenant la solution médicamenteuse est vide, le soluté de rinçage compris dans le compartiment du dessus s'écoule.

Un autre système de perfusion dans lequel un soluté de rinçage s'écoule automatiquement à la fin de la perfusion d'une solution médicamenteuse est décrit dans le document EP-1 722 840. Ce système est formé d'une poche comprenant un premier compartiment contenant la solution médicamenteuse et un deuxième compartiment contenant le soluté de rinçage. Une partie du premier compartiment est prévue au dessus du deuxième compartiment et une autre partie au niveau et/ou en dessous du deuxième compartiment. Une tubulure contenant une valve "bec de canard" relie le premier et le deuxième compartiment, de sorte que, lorsque le premier compartiment est vide ou quasi vide, le soluté de rinçage compris dans le deuxième compartiment s'écoule automatiquement dans le premier compartiment pour le rincer par un effet de dilution.

Bien que ce dernier système fonctionne correctement dans son ensemble, il arrive parfois qu'après passage du soluté de rinçage dans le premier compartiment, il reste de la solution médicamenteuse non diluée dans ce compartiment.

L'invention propose un système de perfusion perfectionné permettant un rinçage optimal afin de respecter précisément le dosage de solution médicamenteuse à perfuser.

A cet effet, l'invention propose un système de perfusion comprenant :
- un ensemble de poches comprenant au moins une première poche et une deuxième poche, ladite première poche contenant ou étant destinée à contenir une première solution, ladite première poche étant pourvue d'au moins un premier tube d'entrée formant orifice d'entrée et d'un deuxième tube de sortie formant orifice de sortie, lesdits premier et deuxième tubes étant chacun arrangés dans un joint périphérique de ladite poche, et ladite deuxième poche contenant ou étant destinée à contenir une deuxième solution, et
- un dispositif de raccordement mettant ou destinés à mettre en communication fluidique la première poche au niveau de son tube d'entrée et la deuxième poche, ledit joint périphérique comprenant une extension faisant saillie vers l'intérieur de ladite première poche de sorte à dévier l'écoulement de la deuxième solution provenant de la deuxième poche du chemin direct d'écoulement entre le tube d'entrée et de sortie.

D'autres objets et avantages apparaîtront au cours de la description qui suit.
La figure 1 représente de façon schématique un système de perfusion selon une première réalisation de l'invention.
La figure 2 représente de façon schématique un système de perfusion selon une deuxième réalisation de l'invention.
La figure 3 représente de façon schématique l'ensemble à poches du système de perfusion de la figure 2, avant connexion d'un dispositif de raccordement.
La figure 4 représente de façon schématique le dispositif de raccordement destiné à être connecté à l'ensemble de poches de la figure 3.
La figure 5 représente de façon schématique une variante d'un ensemble à poches d'un système de perfusion selon l'invention.
La figure 6 représente de façon schématique le système de perfusion de la figure 2 comprenant un dispositif de perfusion.

Les termes "supérieur", "inférieur", "horizontal", "vertical", "au dessus" et "en dessous" sont définis par rapport à la position normale d'utilisation du système de perfusion, c'est-à-dire la position du système au moment où le patient va être perfusé. Les termes "amont" et "aval" sont définis par rapport au sens d'écoulement des solutions dans le système de perfusion.

L'invention concerne un système de perfusion par gravité, notamment destiné à permettre l'administration parentérale de solutions injectables à un patient. En particulier, le système de perfusion permet l'administration d'une première solution injectable contenant un principe actif nécessitant un dosage précis, tel qu'un agent antimitotique ou antibiotique, et d'une deuxième solution injectable permettant de rincer au moins partiellement le système de perfusion afin d'assurer une administration optimale de la première solution injectable.

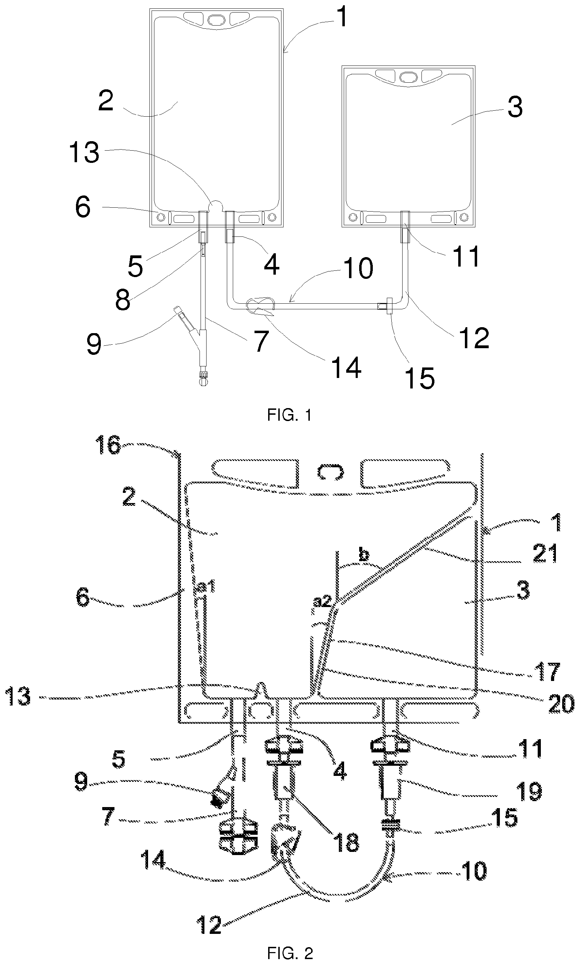

Selon l'invention et en relation avec la figure 1, le système de perfusion 1 comprend un ensemble de poches comprenant au moins une première poche 2 et une deuxième poche 3, ladite première poche 2 contenant ou étant destinée à contenir une première solution et ladite deuxième poche 3 contenant ou étant destinée à contenir une deuxième solution.

Les première et deuxième solutions sont des solutions injectables. Elles sont placées, respectivement, dans les première et deuxième poches 2,3, au moment de la fabrication ou introduites dans le système de perfusion 1 au moment de l'emploi par un orifice d'entrée/sortie de chacune desdites poches.

Par exemple, la première poche 2 contient une solution comprenant un médicament, tel qu'un antibiotique ou un antimitotique, et la deuxième poche 3 contient une solution de rinçage. Des exemples de solution de rinçage comprennent des solutions de chlorure de sodium, glucose et solution de Ringer.

La première poche 2 est pourvue d'au moins un premier tube d'entrée 4 formant orifice d'entrée et d'un deuxième tube de sortie 5 formant orifice de sortie, lesdits premier et deuxième tubes 4,5 étant chacun arrangés dans un joint périphérique 6 de ladite poche 2.

L'orifice d'entrée 4 de la première poche 2 est notamment destiné à permettre l'introduction de la deuxième solution dans la première poche 2.

L'orifice de sortie 5 de la première poche 2 est notamment destiné à permettre l'évacuation de la première et deuxième solution vers le patient à perfuser.

Selon un exemple, le joint périphérique 6 associe entre elles et sur leur périphérie deux feuilles souples formant la première poche 2. Le joint périphérique comprend ainsi deux portions latérales et deux portions transversales.

En variante, la première poche 2 est formée d'une feuille souple repliée sur elle-même, le joint périphérique 6 comprend alors deux portions latérales et une portion transversale.

Selon une autre variante, la poche est formée d'une gaine souple. Dans ce cas, le joint périphérique comprend deux portions latérales ou transversales.

Lorsque la première et la deuxième poche 2,3 forment un premier et deuxième compartiment d'une poche bi-compartimentée 16, comme représentée sur la figure 2 par exemple, une portion du joint périphérique 6 de la première poche 2 s'étend sur le joint de compartimentage 17 de la poche bi-compartimentée 16.

Les tubes d'entrée 4 et de sortie 5 de la première poche 2 sont arrangés soit dans une même portion de joint périphérique 6, soit dans une portion différente.

Avantageusement, les tubes d'entrée 4 et de sortie 5 de la première poche 2 sont disposés sur le même côté de ladite première poche 2, notamment sur une même portion transversale inférieure du joint périphérique 6.

En particulier, l'extension 13 du joint périphérique est arrangée entre le tube d'entrée 4 et le tube de sortie 5 de la première poche 2. Et encore plus avantageusement, l'extension 13 est adjacente au tube d'entrée 4 et au tube de sortie 5.

Le joint périphérique 6 est notamment formé d'un cordon de soudure, obtenu par exemple par soudage thermique ou par haute fréquence. Le cordon de soudure comprend un bord intérieur délimitant le volume intérieur de ladite première poche 2, ladite extension 13 s'étendant à partir dudit bord intérieur du cordon de soudure.

L'extension 13 est notamment réalisée en même temps que le reste du joint périphérique 6, par soudage de la ou les feuilles formant la première poche.

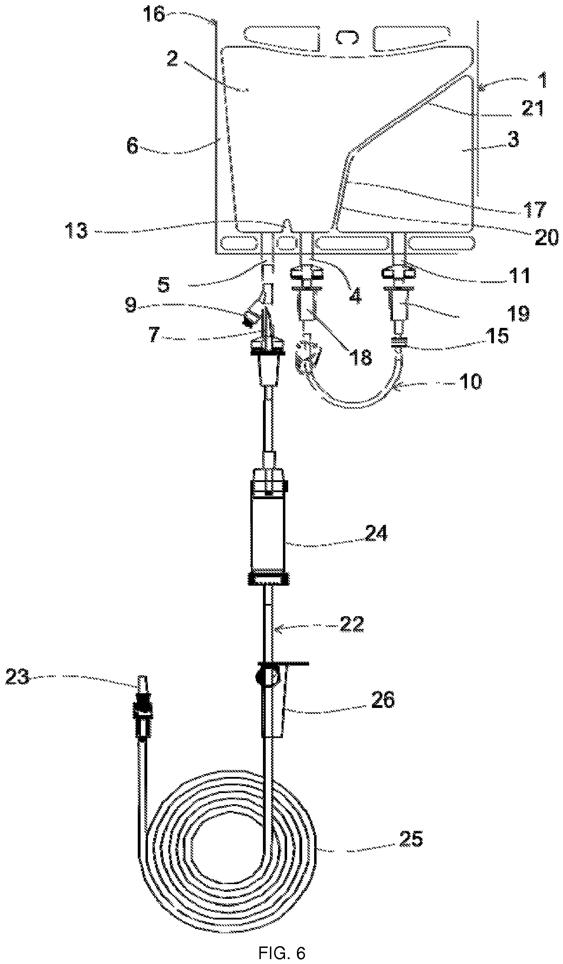

Le système de perfusion 1 comprend en outre un dispositif de perfusion 22 en communication fluidique avec la première poche 2 par l'intermédiaire d'une tubulure de perfusion 7 connectée au tube de sortie 5 de ladite première poche 2 (figure 6). Ce dispositif de perfusion 22 est destiné à être raccordé au circuit veineux du patient, soit directement soit par l'intermédiaire d'un cathéter afin d'administrer au moins la première solution audit patient. Ce dispositif de perfusion 22 est décrit plus en détail ci-dessous en relation avec la figure 6.

En relation avec la figure 1, la tubulure de perfusion 7 ou le tube de sortie 5 de la première poche 2 est pourvu d'un moyen de contrôle sélectif d'ouverture et/ou fermeture de l'écoulement des fluides, tel qu'un ouvre-circuit 8.

Au moment de l'utilisation du système de perfusion, il suffit de casser cet ouvre-circuit 8 pour perfuser la première solution.

En outre, la tubulure de perfusion 7 est pourvue d'un site d'injection 9 qui permet la reconstitution d'une solution médicamenteuse avant la perfusion.

A titre d'exemple, lors de la fabrication du système de perfusion 1, les deux poches 2,3 sont pré-remplies d'une solution injectable de type solution de rinçage. En particulier, les deux poches 2,3 contiennent la même solution. Au moment de l'emploi, un médicament est reconstitué dans la solution contenue dans la première poche 2 à l'aide d'un dispositif de transfert par l'intermédiaire du site d'injection 9 placé sur la tubulure de perfusion 7.

Le système de perfusion 1 comprend en outre un dispositif de raccordement 10 mettant ou destinés à mettre en communication fluidique la première poche 2 au niveau de son tube d'entrée 4 et la deuxième poche 3, au niveau d'un tube de sortie 11 formant orifice de sortie de la deuxième poche 3.

Selon la figure 1, le dispositif de raccordement 10 est formé d'une tubulure de raccordement 12 connectée à une extrémité au tube d'entrée 4 de la première poche 2 et à l'autre extrémité au tube de sortie 11 de la deuxième poche 3.

Lors de l'utilisation d'un tel système de perfusion 1, une fois la totalité ou la majeure partie de la première solution perfusée au patient via le dispositif de perfusion, la deuxième solution contenue dans la deuxième poche 3 s'écoule dans le dispositif de raccordement 10, dans la première poche 2 puis dans le dispositif de perfusion via le tube de sortie 5 de la première poche 2.

Cette étape, dite étape de rinçage, doit notamment permettre de rincer le dispositif de perfusion avec la deuxième solution afin d'éliminer et/ou diluer la première solution.

Lors de l'utilisation du système de perfusion 1, lorsque la première poche 2 se vide, elle a tendance à se collaber, emprisonnant de la solution dans des zones localisées et rendant difficile ou impossible l'étape de rinçage.

Afin d'éviter que la deuxième solution s'écoule dans la première poche 2 sans chasser et/ou diluer suffisamment le reste de première solution de la première poche 2, le joint périphérique 6 dans lequel au moins le tube d'entrée 4 de la première poche 2 est arrangé, comprend une extension 13 faisant saillie vers l'intérieur de ladite première poche 2 de sorte à dévier l'écoulement de la deuxième solution provenant de la deuxième poche 3 du chemin direct d'écoulement entre le tube d'entrée 4 et de sortie 5 de la première poche 2.

Le chemin d'écoulement direct entre le tube d'entrée 4 et de sortie 5 de la première poche 2 est le chemin d'écoulement que suivrait normalement la deuxième solution dans la première poche 2 entre lesdits tubes d'entrée 4 et de sortie 5 lors de l'utilisation du système de perfusion 1, c'est-à-dire lorsque la deuxième solution va être perfusée au patient.

Lorsque le tube d'entrée 4 et de sortie 5 de la première poche 2 sont sur le même bord inférieur de ladite première poche 2, le chemin d'écoulement direct suit sensiblement le bord intérieur du joint périphérique de la première poche. La présence de l'extension 13 entre le tube d'entrée 2 et de sortie 4 empêche la deuxième solution provenant de la deuxième poche 3 de sortir de la première poche 2 sans chasser de la première solution résiduelle.

La longueur et la forme de l'extension 13 sont choisies de sorte que la deuxième solution puisse remonter assez haut dans la première poche 2 lors du rinçage et que la deuxième solution puisse ressortir par le tube de sortie 5 de la première poche 2.

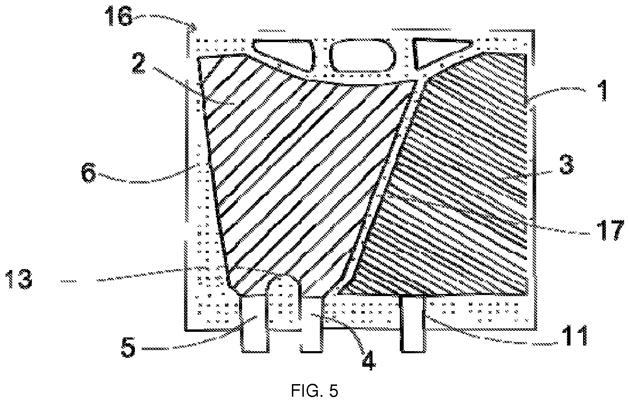

Selon les figures 1 et 5, l'extension 13 du joint périphérique 6 est en forme de demi-cercle. En variante, sur les figures 2,3 et 6, l'extension 13 se rétrécit à partir du joint périphérique 6, formant un cône avec un sommet arrondi.

La tubulure de raccordement 12 est en particulier pourvue d'un moyen de contrôle sélectif d'ouverture / fermeture de l'écoulement des fluides dans ladite tubulure tel qu'un clamp 14 et/ou un ouvre-circuit.

Selon une réalisation particulière, le système de perfusion 1 est apte à réaliser l'écoulement automatique, c'est-à-dire sans intervention humaine, de la deuxième solution contenue dans la deuxième poche 3 vers la première poche 2, lorsque la totalité ou une majeure partie de la solution contenue dans la première poche 2 s'est évacuée de ladite première poche 2.

Pour cela, la tubulure de raccordement 12 est pourvue d'un obturateur réversible d'écoulement 15 apte, en fonction du différentiel de pression de part et d'autre dudit obturateur 15, à permettre ou empêcher l'écoulement de la deuxième solution de la deuxième poche 3 vers la première poche 2.

En particulier, l'obturateur réversible 15 est de type clapet de non retour qu'un excès de pression fait ouvrir momentanément ou fermer si la pression s'exerce dans le sens inverse de l'écoulement de la solution.

Un exemple particulier d'un tel obturateur réversible 15 est un clapet dit "bec de canard" ou valve à seuil de pression.

La position de l'obturateur réversible 15 sur la tubulure de raccordement 12 n'a pas d'influence sur le déclenchement dudit obturateur réversible 15. Pour des raisons pratiques, il est avantageux de le positionner le plus proche de la deuxième poche 3.

Le principe de fonctionnement d'un tel système de perfusion automatique repose sur le principe des "vases communicants" selon lequel les pressions de part et d'autre de l'obturateur réversible 15 et les niveaux des solutions dans les première et deuxième poches 2,3 tendent à s'équilibrer automatiquement.

Dans ce mode automatique, il ne faut pas que l'extension 13 du joint périphérique 6 monte trop haut dans la première poche 2 pour ne pas annuler la différence de hauteur entre les solutions dans les première et deuxième poches 2,3.

Dans un mode particulier de l'invention représenté sur les figures 2-3 et 5-6, la première poche 2 et la deuxième poche 3 forment le premier et le deuxième compartiment d'une poche bi-compartimentée 16, lesdits premier et deuxième compartiments étant juxtaposés et séparés par un joint de compartimentage 17. Le joint de compartimentage 17 est sensiblement vertical.

Dans un exemple particulier, la première poche 2 (ou premier compartiment) est destiné à contenir un volume de première solution, notamment compris entre 25 et 500 mL et la deuxième poche (ou deuxième compartiment), un volume de deuxième solution, notamment compris entre 25 et 150 mL.

Cette configuration du système de perfusion 1 sous forme de poche bi-compartimentée 16 facilite la manipulation du système de perfusion 1.

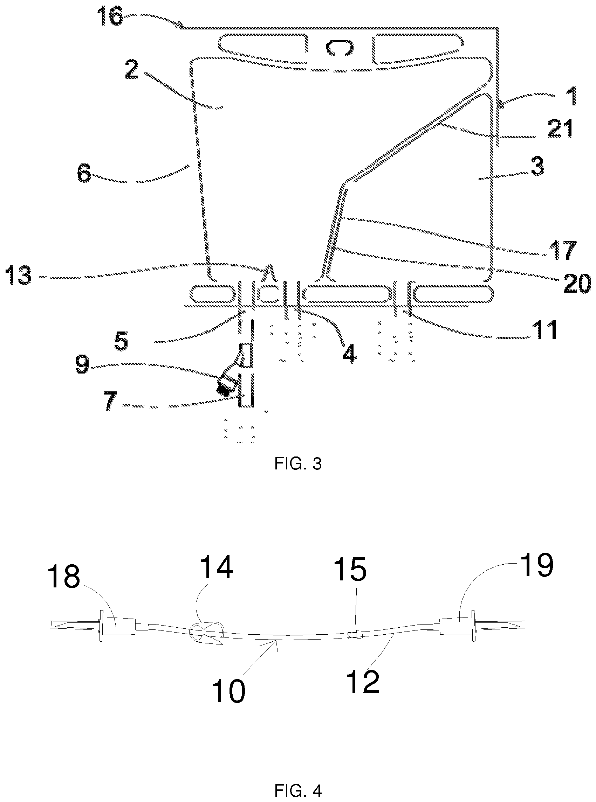

Comme représentée sur la figure 4, la tubulure de raccordement 12 comprend à chacune de ses extrémités, des moyens de connexions 18,19 à l'orifice d'entrée 4 de la première poche 2 et à l'orifice de sortie 11 de la deuxième poche 3, respectivement. Notamment, un tel moyen de connexion est formé d'un perforateur.

Dans une réalisation particulière, le système de perfusion 1 se présente sous la forme d'un kit comprenant d'une part un ensemble de poches par exemple celui de la figure 3 et d'autre part un dispositif de raccordement 10 par exemple celui de la figure 4. Ces deux éléments sont destinés à être connectés au moment de l'utilisation du système de perfusion. Ils sont notamment stérilisés et emballés séparément.

Lors de l'utilisation, il est important, pour éviter le déclenchement précoce de l'écoulement de la deuxième solution, de connecter d'abord la tubulure de raccordement 12 à la deuxième poche 3 puis ensuite à la première poche 2.

Selon une variante de réalisation, le système de perfusion 1 forme un système unitaire en circuit fermé, c'est-à-dire un système prêt à l'emploi, dont les différents éléments le constituant sont pré-connectés. Les éléments étant connectés de fabrication, on évite ainsi une étape de connexion préalablement à l'utilisation du dispositif, qui constitue une manipulation supplémentaire entraînant une perte de temps et un risque de contamination.

Le joint de compartimentage 17 est formé d'un cordon de soudure, notamment réalisé par soudage thermique ou par haute fréquence. Il s'étend à partir de la portion transversale du joint périphérique de la poche bi-compartimentée, comprenant notamment les orifices d'entrée 4 et de sortie 5 de la première poche, et l'orifice de sortie 11 de la deuxième poche.

Selon la figure 5, le joint de compartimentage 17 est incliné par rapport à la verticale pour optimiser l'évacuation des solutions de la première poche.

Dans un exemple de réalisation illustré par exemple sur la figure 5, chacune des deux portions latérales du joint périphérique 6 de la première poche 2 est inclinée vers l'extérieur à partir de la portion transversale du joint périphérique 6 pourvue d'au moins le tube de sortie 5 de la première poche 2.

Notamment, l'angle d'inclinaison a1, a2 des portions latérales de la première poche 2 par rapport à la verticale est compris entre 10 et 20°. Avantageusement, l'angle d'inclinaison a1, a2 est le même pour chacune des deux portions latérales du joint périphérique 6 (figure 2).

Cette configuration inclinée permet d'optimiser l'écoulement de la première et deuxième solution vers le tube de sortie 5 de la première poche 2. On évite ainsi le blocage de la première solution dans les coins de la première poche 2 lors du collabage de celle-ci.

Dans le cas d'une poche bi-compartimentée 16, une portion latérale du joint périphérique 6 de la première poche 2 et le joint de compartimentage 17 sont inclinés par rapport à la verticale.

Selon la figure 5, le joint de compartimentage 17 s'étend entre le bord inférieur et le bord supérieur de la poche bi-compartimentée.

En variante représentée sur la figure 2, le joint de compartimentage 17 s'étend entre le bord inférieur et le bord latéral de la poche bi-compartimentée 16 du côté de la deuxième poche 3. Dans cette configuration, avant la perfusion des solutions, un volume de la première solution dans la première poche 2 est au dessus du volume de la deuxième solution 3 dans la deuxième poche.

Dans une variante de cette réalisation représentée sur la figure 2, le joint de compartimentage 17 comprend une première portion inclinée 20 et une deuxième portion inclinée 21, l'angle d'inclinaison a2 de la première portion 20 du joint de compartimentage 17 par rapport à la verticale étant inférieur à l'angle d'inclinaison b de la deuxième portion 21.

Par exemple, l'angle d'inclinaison a2 de la première portion 20 est compris entre 10 et 20°, en particulier de l'ordre de 12°. L'angle d'inclinaison b de la deuxième portion 21 est compris entre 30 et 40°, en particulier de l'ordre de 35°.

Avec ce joint de compartimentage 17 particulier, le déclenchement de l'obturateur réversible 15 est plus régulier, et l'écoulement de la deuxième solution dans la première poche 2 plus homogène grâce à la colonne d'eau créée dans la première poche en fin d'évacuation de la première solution.

En outre, afin d'augmenter encore la pression à l'intérieur de la première poche et faciliter l'ouverture de l'obturateur réversible 15, la première poche 2 contient un volume d'air. Par ailleurs, ce volume d'air permet d'éviter le collabage de la première poche 2.

Comme représenté sur la figure 6, le système de perfusion 1 comprend en outre un dispositif de perfusion 22. Ce dispositif de perfusion 22 est connecté en pratique au reste du système de perfusion 1 au moment de l'utilisation du système de perfusion.

Le dispositif de perfusion 22 comprend un moyen de raccordement 23 au circuit veineux du patient, telle qu'une aiguille ou un raccord pour cathéter, notamment un raccord mâle de type cône Luer.

Le dispositif de perfusion 22 comprend en outre une chambre compte-gouttes 24, qui constitue un moyen de contrôle du débit de la perfusion. La chambre compte-gouttes 24 est connectée à une extrémité à la tubulure de perfusion 7 et à l'autre extrémité au moyen de raccordement 23 par l'intermédiaire d'une tubulure 25.

Cette chambre compte-gouttes 24 est également appropriée pour amorcer la perfusion de la première solution, c'est-à-dire envoyer de la solution dans le dispositif de perfusion 22.

Un régulateur de débit 26 à roulette pouvant contrôler et stopper le débit de la solution à perfuser est placé sur la tubulure 25 du dispositif de perfusion, notamment en aval de la chambre compte-gouttes 24.

Dans les différentes réalisations du système de perfusion de l'invention, les poches, tubes et tubulures sont réalisés notamment en un matériau thermoplastique souple et stérilisable tel que le polychlorure de vinyle ou une polyoléfine.

En relation avec la figure 6, on décrit maintenant le mode de fonctionnement d'un système de perfusion 1 selon l'invention.

Dans une première phase représentée, correspondant au début de la perfusion, le niveau de la première solution contenue dans la première poche 2 est au dessus du niveau de la deuxième solution contenue dans la deuxième poche 3, et la pression en aval de l'obturateur réversible 15 est supérieure à la pression en amont.

Dans cette première phase, puisque la pression en aval de l'obturateur réversible 15 est supérieure à celle en amont, l'obturateur réversible 15, placé dans la tubulure de raccordement 12, est fermé. Par conséquent, la deuxième solution ne peut pas s'écouler vers la première poche 2 ni la première solution dans la deuxième poche 3. Ainsi, seule la première solution s'écoule dans le dispositif de perfusion 22.

Au cours de la première phase, la première solution s'écoule seule dans le dispositif de perfusion 22, créant une dépression due non seulement à la baisse du niveau de solution mais aussi à l'écrasement de la première poche souple sur elle même (effet de collabage). A un certain niveau, les pressions de part et d'autre de l'obturateur réversible 15 vont s'équilibrer.

Le passage à la deuxième phase est ensuite automatique. Aucune action extérieure n'est nécessaire.

Dans cette deuxième phase, la première solution continuant à s'écouler par gravité dans le dispositif de perfusion 22, la pression en aval de l'obturateur réversible 15 est inférieure à la pression en amont.

L'obturateur réversible 15 est alors ouvert. La deuxième solution contenue dans la deuxième poche 3 s'écoule librement dans la première poche 2. La solution contenue dans la première poche 2 comprenant la première et deuxième solutions s'écoule dans le dispositif de perfusion 22.

La présence d'une extension 13 entre le tube d'entrée 4 et le tube de sortie 5 de la première poche 2 crée une dynamique d'écoulement dans la première poche 2, faisant remonter la deuxième solution dans la première poche 2 et permettant en conséquence un mélange plus efficace des première et deuxième solutions.

Dans le cas particulier où la première solution est une solution médicamenteuse et la deuxième solution est une solution de rinçage, cette deuxième phase constitue l'étape de rinçage.

En effet, dans cette phase, la solution de rinçage contenue dans la deuxième poche 3 s'écoule dans la première poche 2 contenant la solution médicamenteuse, provoquant la dilution de la solution médicamenteuse. La solution qui passe dans la première poche 2 puis dans le dispositif de perfusion 22 est alors une solution médicamenteuse diluée avec la solution de rinçage.

A la fin de la perfusion, la solution médicamenteuse a subit une dilution en série dans la première poche 2 si bien que la quantité de médicament restant en solution est minime, notamment en étant inférieure à 1 % de la quantité présente initialement dans la première poche 2.

Ainsi, le système de perfusion 1 selon l'invention permet l'administration automatique et séquentielle d'une solution de médicament et d'une solution de rinçage. Les derniers millilitres de solution passant dans le dispositif de perfusion 22 comprenant une quantité négligeable de médicament, on peut considérer que le rinçage de la première poche 2 et du dispositif de perfusion 22 est complet à la fin de la perfusion.

Aucun volume d'air ne peut être administré au patient entre la perfusion de la solution médicamenteuse et la solution de rinçage, puisque l'administration des solutions est réalisée de façon séquentielle et sans interruption de l'écoulement de solution dans le dispositif de perfusion 22.

En outre, le passage de la première à la deuxième phase est instantané, ce qui permet d'éviter la stagnation de la solution médicamenteuse dans le système de perfusion 1 et dans l'abord veineux du patient. Ainsi on évite une éventuelle absorption du médicament par les matériaux constituant le système de perfusion et par l'abord veineux du patient, en particulier lors de la perfusion de molécules agressives.

Enfin, puisque les deux solutions sont administrées séquentiellement et automatiquement, le risque d'inversion dans l'ordre d'administration des solutions est éliminé.

Dans un exemple particulier de mise en oeuvre du système de perfusion tel que représenté sur la figure 6 dans lequel les deux poches 2,3 formant deux compartiments sont pré-remplis d'une même solution injectable tel que du chlorure de sodium, on réalise les étapes successives suivantes :
- fermer le clamp 14 du dispositif de raccordement 10,
- connecter le dispositif de raccordement 10 au tube d'entrée 4 de la première poche puis au tube de sortie 11 de la deuxième poche,
- connecter le dispositif de perfusion 22 à la tubulure de perfusion 7,
- réaliser l'amorçage de la première solution dans le dispositif de perfusion 22,
- reconstituer la solution médicamenteuse dans la première poche à l'aide du site d'injection 9, et
- ouvrir le clamp 14 du dispositif de raccordement 10,
- faire s'écouler la première solution dans le dispositif de perfusion 22.

## Revendications

1. Système de perfusion (1) comprenant :
- un ensemble de poches comprenant au moins une première poche (2) et une deuxième poche (3), ladite première poche (2) contenant ou étant destinée à contenir une première solution, ladite première poche (2) étant pourvue d'au moins un premier tube d'entrée (4) formant orifice d'entrée et d'un deuxième tube de sortie (5) formant orifice de sortie, lesdits premier et deuxième tubes étant chacun arrangés dans un joint périphérique (6) de ladite poche, et ladite deuxième poche (3) contenant ou étant destinée à contenir une deuxième solution, et
- un dispositif de raccordement (10) mettant ou destinés à mettre en communication fluidique la première poche (2) au niveau de son tube d'entrée (4) et la deuxième poche (3), **caractérisé en ce que** ledit joint périphérique (6) comprend une extension (13) faisant saillie vers l'intérieur de ladite première poche (2) de sorte à dévier l'écoulement de la deuxième solution provenant de la deuxième poche (3) du chemin direct d'écoulement entre le tube d'entrée (4) et de sortie (5).

2. Système selon la revendication 1, **caractérisé en ce qu'**il comprend en outre un dispositif de perfusion (22) en communication fluidique avec la première poche (2) par l'intermédiaire d'une tubulure de perfusion (7) connectée au tube de sortie (5) de ladite première poche (2).

3. Système selon l'une des revendications 1 ou 2, **caractérisé en ce que** les tubes d'entrée (4) et de sortie (5) de la première poche (2) sont disposés sur le même côté de ladite première poche (2).

4. Système selon l'une quelconque des revendications 1 à 3, **caractérisé en ce que** le joint périphérique (6) est formé d'un cordon de soudure qui comprend un bord intérieur délimitant le volume intérieur de la première poche (2), l'extension (13) s'étendant à partir dudit bord intérieur du cordon de soudure.

5. Système selon l'une quelconque des revendications 1 à 4, **caractérisé en ce que** l'extension (13) du joint périphérique (6) est en forme de demi-cercle.

6. Système selon l'une quelconque des revendications 1 à 4, **caractérisé en ce que** l'extension (13) se rétrécit à partir du joint périphérique (6), formant un cône avec un sommet arrondi.

7. Système selon l'une quelconque des revendications 1 à 6, **caractérisé en ce que** le dispositif de raccordement (10) est formé d'une tubulure de raccordement (12) connectée à une extrémité au tube d'entrée (4) de la première poche (2) et à l'autre extrémité à un tube de sortie (11) formant orifice de sortie de la deuxième poche (3).

8. Système selon la revendication 7, **caractérisé en ce que** la tubulure de raccordement (12) est pourvue d'un obturateur réversible d'écoulement (15) apte, en fonction du différentiel de pression de part et d'autre dudit obturateur, à permettre ou empêcher l'écoulement de la deuxième solution de la deuxième poche (3) vers la première poche (2).

9. Système selon la revendication 8, **caractérisé en ce que** l'obturateur réversible d'écoulement (15) est une valve à seuil de pression.

10. Système selon l'une quelconque des revendications 7 à 9, **caractérisé en ce que** la tubulure de raccordement (12) comprend à chacune de ses extrémités, des moyens de connexions (18,19) à l'orifice d'entrée (4) de la première poche (2) et à l'orifice de sortie (11) de la deuxième poche (3).

11. Système selon la revendication 10, **caractérisé en ce que** les moyens de connexions (18,19) sont formés d'un perforateur.

12. Système selon l'une quelconque des revendications 1 à 11, **caractérisé en ce que** chacune des deux portions latérales du joint périphérique (6) de la première poche (2) est inclinée vers l'extérieur à partir de la portion transversale du joint périphérique (6) pourvue d'au moins le tube de sortie (5) de la première poche (2).

13. Système selon l'une quelconque des revendications 1 à 12, **caractérisé en ce que** la première poche (2) et la deuxième poche (3) forment le premier et le deuxième compartiment d'une poche bi-compartimentée (16), lesdits premier et deuxième compartiments étant juxtaposés et séparés par un joint de compartimentage (17).

14. Système selon la revendication 13, **caractérisé en ce que** ledit joint de compartimentage (17) est formé d'un cordon de soudure.

15. Système selon l'une des revendications 13 ou 14, **caractérisé en ce que** le joint de compartimentage (17) s'étend entre le bord inférieur et le bord latéral de la poche bi-compartimentée (16) du côté de la deuxième poche (3).

16. Système selon la revendication 15, **caractérisé en ce que** le joint de compartimentage (17) comprend une première portion inclinée (20) et une deuxième portion inclinée (21), l'angle d'inclinaison de la première portion (20) du joint de compartimentage (17) par rapport à la verticale étant inférieur à celui de la deuxième portion (21).

## Patentansprüche

1. Infusionssystem (1), umfassend:
- einen Beutelsatz, der mindestens einen ersten Beutel (2) und einen zweiten Beutel (3) umfasst, wobei der erste Beutel (2) eine erste Lösung enthält oder dazu vorgesehen ist, dieselbe zu enthalten, wobei der erste Beutel (2) mit mindestens einem ersten, eine Einlassöffnung bildenden Einlassschlauch (4), und einem zweiten, eine Auslassöffnung bildenden Auslassschlauch (5) versehen ist, wobei der erste und zweite Schlauch jeder in einer Umfangsdichtung (6) des Beutels arrangiert sind, und wobei der zweite Beutel (3) eine zweite Lösung enthält oder dazu vorgesehen ist, dieselbe zu enthalten, und
- eine Anschlussvorrichtung (10), die den ersten Beutel (2) im Bereich seines Einlassschlauches (4), und den zweiten Beutel (3) in Fluidkommunikation bringt oder dazu vorgesehen ist, sie in dieselbe zu bringen, **dadurch gekennzeichnet, dass** die Umfangsdichtung (6) eine Verlängerung (13) umfasst, die zur Innenseite des ersten Beutels (2) hin vorspringt, um das Abfließen der aus dem zweiten Beutel (3) stammenden zweiten Lösung vom direkten Abflussweg zwischen dem Einlass- (4) und Auslassschlauch (5) abzulenken.

2. System nach Anspruch 1, **dadurch gekennzeichnet, dass** es weiter eine Infusionsvorrichtung (22) umfasst, die mittels einer mit dem Auslassschlauch (5) des ersten Beutels (2) verbundenen Infusionsleitung (7) mit dem ersten Beutel (2) in Fluidkommunikation steht.

3. System nach einem der Ansprüche 1 oder 2, **dadurch gekennzeichnet, dass** der Einlass- (4) und Auslassschlauch (5) des ersten Beutels (2) auf derselben Seite des ersten Beutels (2) angeordnet sind.

4. System nach einem der Ansprüche 1 bis 3, **dadurch gekennzeichnet, dass** die Umfangsdichtung (6) von einer Schweißnaht gebildet wird, die eine Innenkante umfasst, welche das Innenvolumen des ersten Beutels (2) begrenzt, wobei sich die Verlängerung (13) ab der Innenkante der Schweißnaht erstreckt.

5. System nach einem der Ansprüche 1 bis 4, **dadurch gekennzeichnet, dass** die Verlängerung (13) der Umfangsdichtung (6) in Form eines Halbkreises ist.

6. System nach einem der Ansprüche 1 bis 4, **dadurch gekennzeichnet, dass** sich die Verlängerung (13) ab der Umfangsdichtung (6) verjüngt, wobei sie einen Kegel mit einer abgerundeten Spitze bildet.

7. System nach einem der Ansprüche 1 bis 6, **dadurch gekennzeichnet, dass** die Anschlussvorrichtung (10) von einer Anschlussleitung (12) gebildet wird, die an einem Ende mit dem Einlassschlauch (4) des ersten Beutels (2), und am anderen Ende mit einem Auslassschlauch (11) verbunden ist, der eine Auslassöffnung des zweiten Beutels (3) bildendet.

8. System nach Anspruch 7, **dadurch gekennzeichnet, dass** die Verbindungsleitung (12) mit einer reversiblen Abflusssperre (15) versehen ist, die in der Lage ist, abhängig von der Druckdifferenz auf beiden Seiten der Sperre das Abfließen der zweiten Lösung aus dem zweiten Beutel (3) zum ersten Beutel (2) hin zu ermöglichen oder zu verhindern.

9. System nach Anspruch 8, **dadurch gekennzeichnet, dass** die reversible Abflusssperre (15) ein Ventil mit Druckschwelle ist.

10. System nach einem der Ansprüche 7 bis 9, **dadurch gekennzeichnet, dass** die Anschlussleitung (12) an jedem ihrer Enden Mittel zum Verbinden (18, 19) mit der Einlassöffnung (4) des ersten Beutels (2) und mit der Auslassöffnung (11) des zweiten Beutels (3) umfasst.

11. System nach Anspruch 10, **dadurch gekennzeichnet, dass** die Verbindungsmittel (18, 19) von einem Dorn gebildet werden.

12. System nach einem der Ansprüche 1 bis 11, **dadurch gekennzeichnet, dass** jeder der zwei Seitenabschnitte der Umfangsdichtung (6) des ersten Beutels (2) ab dem Querabschnitt der Umfangsdichtung (6), der mit mindestens dem Auslassschlauch (5) des ersten Beutels (2) versehen ist, zur Außenseite hin geneigt ist.

13. System nach einem der Ansprüche 1 bis 12, **dadurch gekennzeichnet, dass** der erste Beutel (2) und der zweite Beutel (3) die erste und die zweite Kammer eines Zweikammerbeutels (16) bilden, wobei die erste und zweite Kammer nebeneinanderliegen und über eine Kammertrenndichtung (17) getrennt sind.

14. System nach Anspruch 13, **dadurch gekennzeichnet, dass** die Kammertrenndichtung (17) von einer Schweißnaht gebildet wird.

15. System nach einem der Ansprüche 13 oder 14, **dadurch gekennzeichnet, dass** sich die Kammertrenndichtung (17) zwischen der Innenkante und der Seitenkante des Zweikammerbeutels (16) auf der Seite des zweiten Beutels (3) erstreckt.

16. System nach Anspruch 15, **dadurch gekennzeichnet, dass** die Kammertrenndichtung (17) einen ersten geneigten Abschnitt (20) und einen zweiten geneigten Abschnitt (21) umfasst, wobei der Neigungswinkel des ersten Abschnitts (20) der Kammertrenndichtung (17), auf die Vertikale bezogen, kleiner ist als jener des zweiten Abschnitts (21) .

## Claims

1. Infusion system (1) comprising:
- a set of pouches comprising at least a first pouch (2) and a second pouch (3), said first pouch (2) containing or being intended to contain a first solution, said first pouch (2) being provided with at least one first inlet tube (4) acting as an inlet orifice and a second outlet tube (5) acting as an outlet orifice, said first and second tubes being each arranged in a peripheral seal (6) of said pouch, and said second pouch (3) containing or being intended to contain a second solution, and
- a connection device (10) placing or intended to place in fluidic communication the first pouch (2) at the level of the inlet tube (4) thereof and the second pouch (3), **characterised in that** said peripheral seal (6) comprises an extension (13) projecting towards the inside of said first pouch (2) so as to divert the flow of the second solution from the second pouch (3) from the direct flow path between the inlet (4) and outlet (5) tube.

2. System according to claim 1, **characterised in that** it further comprises an infusion device (22) in fluidic communication with the first pouch (2) via an infusion nozzle (7) connected to the outlet tube (5) of said first pouch (2).

3. System according to one of claims 1 or 2, **characterised in that** the inlet (4) and outlet (5) tubes of the first pouch (2) are arranged on the same side of said first pouch (2).

4. System according to any one of claims 1 to 3, **characterised in that** the peripheral seal (6) is formed from a weld seam which comprises an internal edge delimiting the internal volume of the first pouch (2), the extension (13) extending from said internal edge of the weld seam.

5. System according to any one of claims 1 to 4, **characterised in that** the extension (13) of the peripheral seal (6) is in the shape of a semi-circle.

6. System according to any one of claims 1 to 4, **characterised in that** the extension (13) contracts from the peripheral seal (6), forming a cone with a rounded vertex.

7. System according to any one of claims 1 to 6, **characterised in that** the connection device (10) is formed from a connection nozzle (12) connected at one end to the inlet tube (4) of the first pouch (2) and at the other end to an outlet tube (11) acting as an outlet orifice of the second pouch (3).

8. System according to claim 7, **characterised in that** the connection nozzle (12) is provided with a reversible flow valve (15) suitable, according to the pressure differential on either side of said valve, for enabling or preventing the flow of the second solution from the second pouch (3) to the first pouch (2).

9. System according to claim 8, **characterised in that** the reversible flow valve (15) is a pressure threshold valve.

10. System according to any one of claims 7 to 9, **characterised in that** the connection nozzle (12) comprises at each of the ends thereof, connection means (18, 19) to the inlet orifice (4) of the first pouch (2) and to the outlet orifice (11) of the second pouch (3).

11. System according to claim 10, **characterised in that** the connection means (18, 19) are formed from a perforator.

12. System according to any one of claims 1 to 11, **characterised in that** each of the two lateral portions of the peripheral seal (6) of the first pouch (2) is inclined outwards from the transverse portion of the peripheral seal (6) provided with at least the outlet tube (5) of the first pouch (2).

13. System according to any one of claims 1 to 12, **characterised in that** the first pouch (2) and the second pouch (3) form the first and the second compartment of a two-compartment pouch (16), said first and second compartments being juxtaposed and separated by a compartmentalisation seal (17).

14. System according to claim 13, **characterised in that** said compartmentalisation seal (17) is formed from a weld seam.

15. System according to one of claims 13 or 14, **characterised in that** the compartmentalisation seal (17) extends between the lower edge and the lateral edge of the two-compartment pouch (16) on the side of the second pouch (3).

16. System according to claim 15, **characterised in that** the compartmentalisation seal (17) comprises a first inclined portion (20) and a second inclined portion (21), the angle of inclination of the first portion (20) of the compartmentalisation seal (17) with respect to the vertical being less than that of the second portion (21).
